# EUROPEAN PATENT APPLICATION

(11) **EP 2 460 456 A1**
(43) Date of publication of application: **06.06.2012**
(21) Application number: 11171063.8
(22) Date of filing: 22.06.2011
(51) Int. Cl.: A61B 1/00, A61B 1/04

(54) **Expandable capsule endoscope**

(30) Priority: 02.12.2010 TW 099141846
(71) Applicant: Chung-Shan Institute of Science and Technology, Armaments, Bureau, Ministry of National Defense, Taoyuan County 325, Chinese Taipei (TW)
(72) Inventor: Yeh, Chuen-Tai, 325 Taoyuan County (TW); Lin, Tah-Yeong, 325 Taoyuan County (TW); Wu, Shsien-Ming, 325 Taoyuan County (TW); Huang, Sin-Ming, 325 Taoyuan County (TW); Cho, Kun-Liang, 325 Taoyuan County (TW)
(74) Representative: Lang, Christian

(57) **Abstract**

An expandable capsule endoscope includes a shell, a membrane and a bag. Inside the shell are a first compartment and a second compartment, and the membrane is disposed between the first compartment and the second compartment. The bag is connected outside the shell. There is a first substance and a second substance respectively disposed in the first compartment and the second compartment. When the membrane is ruined and broken by a heating energy, the first substance and the second substance react to produce a gas filling the bag. The expandable capsule endoscope is applicable to examine an organ with wider diameters and advantageous to capture images of the organ thus providing better reliability and utility.

## Description

### BACKGROUND OF INVENTION

### 1. FIELD OF INVENTION

The present invention relates to a capsule endoscope and, more particularly, to an expansible capsule endoscope.

### 2. RELATED PRIOR ART

Endoscopes are used to scrutinize the interior of the alimentary canal. A conventional endoscope includes a lens attached to a long, thin, flexible tube and image-transmission element such as an optical fiber inserted in the tube and connected to the lens. The tube is inserted into the alimentary canal so that the interior of the alimentary canal is visible through the lens. The image of the interior of the alimentary canal is transmitted to a computer through the fiber optical. The image is processed in the computer and shown on a display for review. The alimentary canal could however be too long and winding for smooth insertion of the conventional endoscope. Hence, the patient often feels uncomfortable.

To overcome the foregoing problem, there have been devised capsule endoscopes. A capsule endoscope includes a capsule-like shell to contain a light source, an image sensor, a controller chip, a wireless transmitter and a power supply. In use, the capsule endoscope is swallowed by a patient. While traveling through the alimentary canal of the patient, the capsule endoscope takes and transmits the image of the interior of the alimentary canal to a computer equipped with a wireless receiver located outside the patient. The capsule endoscope will eventually be released from the patient. The use of the capsule endoscopes is convenient compared with the use of the conventional endoscopes. Therefore, a capsule endoscope is often used instead of a conventional endoscope.

A capsule endoscope can effectively be used to scrutinize the small intestine since the former smoothly travels through the latter without much rolling. However, it has not been a successful attempt to use the capsule endoscope to scrutinize the large intestine, which is generally more vulnerable to disease than the small intestine, because the capsule endoscope inevitably rolls in the large intestine.

To overcome the foregoing problem, there have been devised expansible capsule endoscopes. An expansible capsule endoscope includes an inflatable bag and a remotely controllable solenoid valve. When the expansible capsule endoscope reaches the large intestine, the solenoid valve is opened in a remote manner to allow two materials to react with each other to produce gas to inflate the bag. Expanded, the bag gently abuts the interior of the large intestine and stabilizes itself in the large intestine so that it smoothly travels through the large intestine to clearly take and transmit the image of the interior of the large intestine to the computer.

The solenoid valve is however too big to allow the expansible capsule endoscope to smoothly travel through the small intestine. Furthermore, it is difficult to operate the solenoid valve in the remote manner because the solenoid valve might be too tight to open or too slack to shut.

The present invention is therefore intended to obviate or at least alleviate the problems encountered in prior art.

### SUMMARY OF INVENTION

It is the primary objective of the present invention to provide a reliable expansible capsule endoscope.

To achieve the foregoing objectives, the expansible capsule endoscope includes a shell, a membrane, two materials, a bag and a power element. The shell includes at least one aperture defined therein. The membrane is located in the shell, thus dividing the space of the shell into a first space and a second space. The first material is contained in the first space. The second material is contained in the second space. The bag is connected to the shell so that the bag is in communication with the shell through the aperture. The power element is located in the shell near the membrane so that the power element is operable to produce heat to melt and break the membrane to allow the first material to react with the second material to produce gas to inflate the bag.

In an aspect, the expansible capsule endoscope may further includes a wireless receiver for receiving a wireless signal for turning on the power element and a power supply for energizing the wireless receiver.

In another aspect, the membrane is made of latex.

In another aspect, one of the first and second materials is acid while the other material is alkali.

In another aspect, the acid is acetic acid while the alkali is sodium bicarbonate.

Other objectives, advantages and features of the present invention will be apparent from the following description referring to the attached drawings.

### BRIEF DESCRIPTION OF DRAWINGS

The present invention will be described via detailed illustration of the preferred embodiment referring to the drawings wherein:
FIG. 1 is a cross-sectional view of an expansible capsule endoscope according to the preferred embodiment of the present invention; and
FIG. 2 is a cross-sectional view of the expansible capsule endoscope in an expanded position other than the original position shown in FIG. 1.

### DETAILED DESCRIPTION OF PREFERRED EMBODIMENT

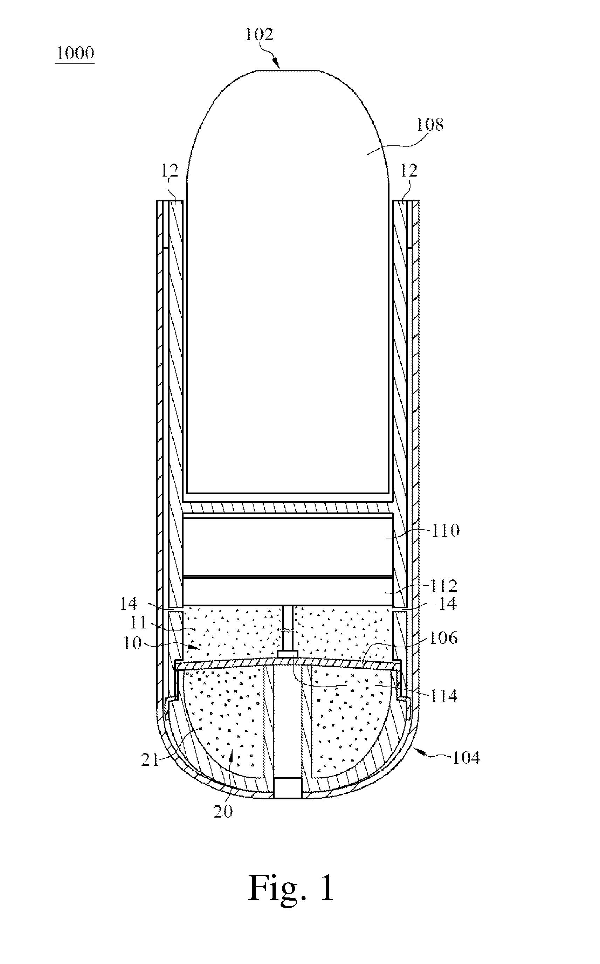

Referring to FIG. 1, an expansible capsule endoscope 1000 according to the preferred embodiment of the present invention is shown. The expansible capsule endoscope 1000 includes a first shell 102, a second shell 12 and a bag 104. An image unit 108 is located in it the first shell 102.

The image unit 108 is used to take and transmit the image of the interior of the alimentary canal, mouth, nose, anus or vagina. The image unit 108 includes a light source, optical elements and a wireless unit. The light source is used to cast light onto the interior of the organ. The optical elements include, but not limited to, a lens assembly and an image sensor. The optical elements are used to convert light reflected from the interior of the organ to image-related data. The wireless unit may be an RF unit for example. The wireless unit is used to transmit the image-related data to a corresponding wireless unit used in a computer located outside a patient. The image unit 108 is not the spirit of the present invention and therefore will not be described in detail.

The second shell 12 includes a cylinder, a cap and a membrane 106. The membrane 106 is made of latex for example. The cylinder of the second shell 12 includes a partition formed on an internal side thereof, thus dividing the cylinder of the second shell 12 into two sections. The first section of the cylinder of the second shell 12 is used to contain the first shell 102. The second section of the cylinder of the second shell 12 includes a space 10 defined therein and apertures 14 in communication with the space 10.

A power supply 110, a wireless receiver 112, a power element 114 and a first material 11 are contained in the space 10. The power supply 110 includes two batteries of 1.5 volts for example. The power supply 110 is used to energize the wireless receiver 112, the power element 114 and the image unit 108. The wireless receiver 112 is used to receive a wireless signal from a related wireless transmitter located in the computer located outside the patient. The wireless receiver 112 may be an RF or magnetic receiver for example. The power element 114 is used to produce heat for melting and breaking the membrane 106. The power element 114 may be an electric heater or any other power element made of an electro-thermal material.

The cap of the second shell 12 includes a space 20 defined therein for containing a second material 21. The cap of the second shell 12 is connected to the second section of the cylinder of the second shell 12 after the membrane 106 is located between them. Thus, the space 20 is separated from the space 10 by the membrane 106. Hence, the second material 21 is separated from the first material 11 by the membrane 106. The membrane 106 is in contact with or at least in the vicinity of the power element 114.

The first material 11 is acid while the second material 21 is alkali, or the first material 11 is alkali while the second material 21 is acid. For example, the acid may be acetic acid while the alkali may be sodium bicarbonate. The first material 11 can react with the second material 21 to produce a large amount of gas to expand the bag 104.

The bag 104 is made of an elastic material so that it can be inflated. The bag 104 includes an edge connected to an edge of the second shell 12 so that there is a space defined between the bag 104 and the second shell 12. The connection of the bag 104 to the second shell 12 is airtight. The space defined in the bag 104 is in communication with the space 10 through the apertures 14.

In operation, the expansible capsule endoscope 1000 is swallowed by the patient so that the expansible capsule endoscope 1000 travels in and along the patient's alimentary canal. As the expansible capsule endoscope 1000 travels in and along the small intestine, the image unit 108 takes and transmits the image of the interior of the small intestine to the computer.

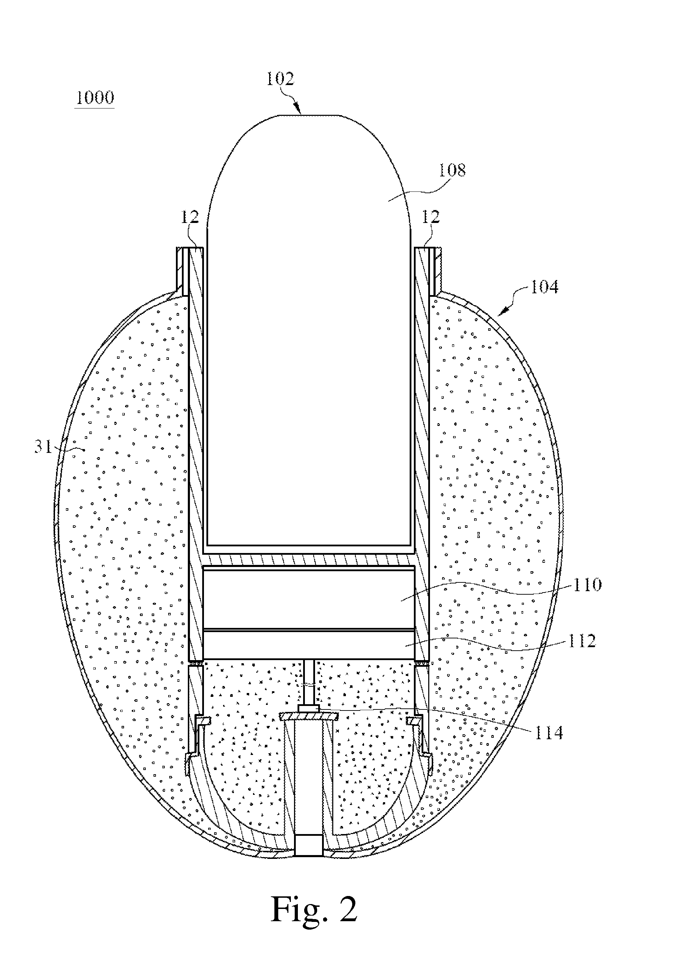

As the expansible capsule endoscope 1000 reaches the large intestine, a wireless signal is sent to the wireless receiver 112 from the related wireless transmitter located in the computer located outside the patient. On the moment of the receipt of the wireless signal, the wireless receiver 112 turns on the power element 114 to produce heat to melt and break the membrane 106. Thus, as shown in FIG. 2, the membrane 106 is broken to allow the first material 11 to react with the second material 21 to produce a large amount of gas 31. The gas 31 goes into the space defined between the second shell 12 and the bag 104 through the apertures 14 to expand the bag 104. The expanded bag 104 is in gentle contact with the interior of the large intestine to cause the expansible capsule endoscope 1000 to travel smoothly in and along the large intestine to clearly take the image of the interior of the large intestine.

A first medium is provided on a first side of the membrane 106, which is in contact with or at least located near the power element 114 while a second medium is provided on a second side of the membrane 106. The thermal conductivity of the first medium is higher than the thermal conductivity of the second medium. Thus, the heat produced by the power element 114 is substantially confined to the first side of the membrane 106 so that the membrane 106 is effectively heated, melted and broken. The second medium may be air for example.

As discussed above, the expansible capsule endoscope 1000, in its original shape, stabilizes itself in the small intestine and clearly takes the image of the interior of the small intestine. Moreover, the expansible capsule endoscope 1000, in the expanded state, stabilizes itself in the large intestine and clearly takes the image of the interior of the large intestine. The power element 114 precisely and reliably operates without having to move any elements. Therefore, the expansible capsule endoscope 1000 is reliable.

The present invention has been described via the detailed illustration of the preferred embodiment. Those skilled in the art can derive variations from the preferred embodiment without departing from the scope of the present invention. Therefore, the preferred embodiment shall not limit the scope of the present invention defined in the claims.

## Claims

1. An expansible capsule endoscope including:
a shell 12 including at least one aperture 14 defined therein;
a membrane 106 located in the shell 12, thus dividing the space of the shell 12 into a first space 10 and a second space 20;
a first material 11 contained in the first space 10;
a second material 21 contained in the second space 20;
a bag 104 connected to the shell 12 so that the bag 104 is in communication with the shell 12 through the aperture 14; and
a power element 114 located in the shell 12 near the membrane 106 so that the power element 114 is operable to produce heat to melt and break the membrane 106 to allow the first material 11 to react with the second material 21 to produce gas 31 to inflate the bag 104.

2. The expansible capsule endoscope according to claim 1, further including:
a wireless receiver 112 for receiving a wireless signal for turning on the power element 104; and
a power supply 110 for energizing the wireless receiver 112.

3. The expansible capsule endoscope according to claim 1, wherein the membrane 106 is made of latex.

4. The expansible capsule endoscope according to claim 1, wherein one of the first and second materials is acid while the other material is alkali.

5. The expansible capsule endoscope according to claim 4, wherein the acid is acetic acid.

6. The expansible capsule endoscope according to claim 4, wherein the alkali is sodium bicarbonate.
